**Europäisches Patentamt**

**European Patent Office**

**Office européen des brevets**

(19)

(11) Veröffentlichungsnummer: **0 141 326**
**B1**

(12) # EUROPÄISCHE PATENTSCHRIFT

(45) Veröffentlichungstag der Patentschrift:
**10.08.88**

(51) Int. Cl.⁴: **C 07 K 15/00,** A 61 K 37/02,
**G 01 N 33/68**

(21) Anmeldenummer: **84112372.2**

(22) Anmeldetag: **13.10.84**

(54) **Neues Protein PP20, Verfahren zu seiner Gewinnung sowie seine Verwendung.**

(30) Priorität: **22.10.83 DE 3338480**

(43) Veröffentlichungstag der Anmeldung:
**15.05.85 Patentblatt 85/20**

(45) Bekanntmachung des Hinweises auf die Patenterteilung:
**10.08.88 Patentblatt 88/32**

(84) Benannte Vertragsstaaten:
**AT BE CH DE FR GB IT LI LU NL SE**

(56) Entgegenhaltungen:
**EP-A-0 033 000**
**EP-A-0 060 491**
**EP-A-0 101 603**

(73) Patentinhaber: **BEHRINGWERKE Aktiengesellschaft,
Postfach 1140, D-3550 Marburg 1 (DE)**

(72) Erfinder: **Bohn, Hans, Dr., Oberer Eichweg 26,
D-3550 Marburg (DE)**
Erfinder: **Winckler, Wilhelm, Untere Bergstrasse
21, D-3556 Wenkbach (DE)**

(74) Vertreter: **Meyer- Dulheuer, Karl- Hermann, Dr.,
HOECHST Aktiengesellschaft Zentrale
Patentabteilung Postfach 80 03 20, D-6230
Frankfurt/Main 80 (DE)**

EP 0 141 326 B1

## Beschreibung

Die Erfindung betrifft ein neues Protein $PP_{20}$, ein Verfahren zu seiner Anreicherung und Gewinnung aus einem Extrakt menschlicher Plazenten sowie seine Verwendung.

In Extrakten aus menschlichen Plazenten wurde bereits eine Reihe löslicher Proteine, die aus diesem Gewebe stammen, nachgewiesen (Bohn, H., La Ricerca Clin. Lab. 12, 221, 1982).

In der vorliegenden Patentanmeldung wird die Isolierung und Charakterisisierung eines neuen löslichen Proteins, genannt $PP_{20}$ beschrieben.

Gegenstand der Erfindung ist das Protein $PP_{20}$, gekennzeichnet durch

a) eine elektrophoretische Beweglichkeit bei pH 8,6, die etwas größer ist als die von Albumin,

b) einen isoelektrischen Punkt Von $4,65 \pm 0,1$,

c) einen Sedimentationskoeffizienten $s_{20,w}$ von $4,1 \pm 0,1$ S,

d) ein im Natriumdodecylsulfat (SDS)-haltigen Polyacrylamidgel elektrophoretisch bestimmtes Molekulargewicht von $50\ 000 \pm 10\ 000$, wobei die Moleküle von $PP_{20}$ aus anscheinend identischen Untereinheiten, die ein Molekulargewicht von $27\ 000 \pm 3\ 000$ haben und nicht-kovalent zusammengehalten werden, aufgebaut sind,

e) einen Extinktionskoeffizienten $E^{1\%}_{1cm}$ (280 nm) von $9,5 \pm 0,6$,

f) einen Kohlenhydratanteil von $3,0 \pm 1,3$ %, darunter Mannose $0,14 \pm 0,05$ %, Fucose $0,13 \pm 0,05$ %, Galaktose, $0,61 \pm 0,2$ %, Glukose $0,39 \pm 0,2$ %, N-Acetyl-Glukosamin $0,95 \pm 0,3$ %, N-Acetyl-Galaktosamin $0,18 \pm 0,1$ %, N-Acetyl-Neuraminsäure $0,6 \pm 0,4$ % und

g) die folgende Aminosäurenzusammensetzung:

| Aminosäure | (Reste pro 100 Reste) Mol-% | Variations- koeffizient |
|---|---|---|
| Lysin | 5,36 | 0,26 |
| Histidin | 3,10 | 7,30 |
| Arginin | 3,39 | 0,63 |
| Asparaginsäure | 11,45 | 1,05 |
| Threonin | 8,63 | 1,31 |
| Serin | 3,70 | 1,34 |
| Glutaminsäure | 9,65 | 1,61 |
| Prolin | 5,64 | 6,40 |
| Glycin | 6,96 | 1,12 |
| Alanin | 4,76 | 1,49 |
| Cystin 1/2 | 2,86 | 2,47 |
| Valin | 6,22 | 0,23 |
| Methionin | 1,87 | 1,51 |
| Isoleucin | 5,76 | 3,44 |
| Leucin | 11,76 | 2,65 |
| Tyrosin | 2,55 | 3,33 |
| Phenylalanin | 4,12 | 1,55 |
| Tryptophan | 2,17 | 1,30 |

Zur Erläuterung der kennzeichnenden Merkmale des Gewebe-proteins sei folgendes ausgeführt:

Die elektrophoretische Beweglichkeit wurde mit dem Gerät Microzone R 200 von Beckman Instruments auf Zelluloseazetatfolien (Firma Sartorius) unter Verwendung von Natriumdiäthylbarbiturat-Puffer, pH 8,6, bestimmt.

Der isoelektrische Punkt wurde mit einer Säule (440 ml) der Firma LKB, Stockholm, festgestellt. Das Sogenannte Ampholin®-Gemisch hatte bei der Untersuchung des Glykoproteins einen pH-Bereich von 4,0 bis 6,0.

Der Sedimentationskoeffizient wurde in einer analytischen Ultrazentrifuge der Firma Beckman (Spinco-Apparatur, Modell E) bei 60 000 UpM in Doppelsektorzellen mit Hilfe der UV-Scanner Technik bei 280 nm bestimmt. Als Lösungsmittel diente ein 0,05 M Phosphatpuffer (pH 6,8), der 0,2 Mol/l NaCl enthielt. Die Proteinkonzentration wurde auf eine optische Dichte von etwa 3 eingestellt. Der Sedimentationskoeffizient wurde auf die Basis von Wasser bei 20°C umgerechnet.

Zur elektrophoretischen Bestimmung des Molekulargewichtes im SDS-PAA-Gel wurde ein Gel mit 7,5 g/100 ml Polyacrylamid (PAA), das 0,1 g/100 ml Natriumdodecylsulfat (SDS) enthielt, verwendet.

Zur Untersuchung auf Untereinheiten ist das Protein vor dem Auftragen in einer Lösung, die 1 g/100 ml SDS und gegebenenfalls 1 g/100 ml Merkaptoethanol enthielt, 10 Minuten auf 60°C erhitzt worden. Als Vergleichssubstanz dienten humanes Plazentalaktogen (HPL) und Human-Albumin sowie dessen Aggregate.

Zur Bestimmung des Extinktionskoeffizienten wurde die Substanz 0,10 %-ig (g/100 ml) in destilliertem Wasser gelöst.

Die Analyse der Kohlenhydrate wurde wie folgt durchgeführt: Nach Hydrolyse der glykosidischen Bindungen wurden die freigesetzten Neutralzucker als Boratkomplexe über eine Anionenaustauschersäule getrennt (Y. C. Lee et al., Anal. Biochem. 27, 567, 1969) im Eluat durch Zumischung von Cu(I)-bicinchoninatreagenz (K. Mopper und M. Gindler, Anal. Biochem. 56, 440, 1973) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI, 463-465, 1963) ermittelt worden.

Die Aminosäurenanalyse wurde nach S. Moore, D. H. Spackman, W. H. Stein, Anal. Chem. 30, 1185, 1958, unter Verwendung des Flüssigkeitschromatographen Multichrom B der Firma Beckman durchgeführt. 1/2-Cystin wurde nach Oxydation der Protein mit Perameisensäure (S. Moore et al., Anal. Chem. 30, 1185, 1958) und nachfolgender Chromatographie (S. Moore, J. Biol. Chem., 238, 235, 1963) als Cysteinsäure bestimmt. Der Tryptophangehalt ist mit der direkten photometrischen Bestimmung nach H. Edelhoch, Biochemistry 6, 1948, 1967), ermittelt worden.

$PP_{20}$ hat folgende Eigenschaften, die sich in einem Verfahren zu seiner Isolierung verwenden lassen, indem diesen Eigenschaften entsprechende Maßnahmen getroffen werden:
1)	Mit Ammoniumsulfat wird es bei pH 7 und 30-70 % Sättigung aus wäßrigen Lösungen gefällt;
2)	Mit wasserlöslichen Acridinbasen, zum Beispiel 2-Äthoxi-6,9-diaminoacridinlactat wird es bei pH-Werten zwischen 4 - 7 und einer Konzentration der Base von 0,2 bis 0,4 g/100 ml präzipitiert;
3)	Bei der elektrophoretischer Auftrennung wandert es bei pH 8,6 etwas schneller als Albumin;
4)	Bei der isoelektrischen Fokussierung erscheint es im pH-Bereich von 4,55 bis 4,75 mit Maximum bei 4,65;
5)	Bei der Gelfiltration mit Sephadex® oder Acrylamidagarose verhält es sich wie Proteine mit Molekulargewichten von 30 000 bis 70 000;
6)	Es wird in verdünnten Salzlösungen bei einer Leitfähigkeit von etwa 0 - 2 mS im Gegensatz zu den meisten anderen Proteinen nicht an Bentonit (Kalziumbentonit) adsorbiert;
7)	Es läßt sich an schwach basische Ionenaustauscher wie beispielsweise DEAE-Cellulose oder DEAE-Sephadex bei einer Leitfähigkeit von etwa 0 - 2 mS und einem pH-Wert von etwa pH 7 bis 9 binden und mit konzentrierteren Salzlösungen, beispielsweise 1 - 5 g NaCl auf 100 ml enthaltende Lösungen, eluieren;
8)	Es kann aus einer wäßrigen Lösung durch Immunadsorption angereichert und isoliert werden.

Gegenstand der Erfindung ist deshalb ferner ein Verfahren zur Gewinnung des $PP_{20}$, dadurch gekennzeichnet, daß ein Extrakt aus menschlichen Plazenten unter Ausnutzung der obenstehenden Eigenschaften fraktioniert wird.

Gegenstand der Erfindung ist demnach ein Verfahren zur Gewinnung oder Anreicherung des $PP_{20}$, dadurch gekennzeichnet, daß ein aus Plazenten mit einer verdünnten Salz-oder Pufferlösung gewonnener Extrakt zwei oder mehr der folgenden Maßnahmen unterworfen wird:

a)	Fällung des Proteins $PP_{20}$ mit Ammoniumsulfat im pH-Bereich von 5 bis 8 und bei 30-70 % Sättigung;
b)	Fällung des Proteins $PP_{20}$ mit einer wasserlöslichen Acridinbase bei einem pH-Wert zwischen 4 und 7 und einer Konzentration der Base von 0,2 - 0,4 g/100 ml;
c)	präparative Zonenelektrophorese, wobei die Proteinfraktion, die etwas schneller als Albumin wandert, gewonnen wird;
d)	Gelfiltration oder Ultrafiltration, wobei Proteine im Molekulargewichtsbereich von 30.000 bis 70.000 gewonnen werden;
e)	isoelektrische Fokussierung, wobei die Proteine im pH-Bereich von 4,55 bis 4,75 gewonnen werden;
f)	Adsorption von Begleitproteine an Bentonit (Bentonit A, Kalziumbentonit, Erbslöh & Co., Geisenheim a. Rhein), wobei $PP_{20}$ in Lösung bleibt;
9)	Adsorption des Proteins $PP_{20}$ an einem schwach basischen Ionenaustauscher und Elution dieses Proteins;
h)	immunadsorptive Anreicherung.

Neben Ammoniumsulfat können selbstverständlich auch andere in der präparativen Biochemie üblicherweise eingesetzte Neutralsalze zur Ausfällung des $PP_{20}$ verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Entsprechend seinem elektrophoretischen Verhalten, seinem isoelektrischen Punkt und seinem Molekulargewicht, sind zur Isolierung des Proteines auch andere Maßnahmen brauchbar, die geeignet sind, ein Protein mit den angegebenen Eigenschaften von anderen Proteinen abzutrennen. Hierzu können die verschiedenen Methoden der präparativen Elektrophorese, der isoelektrischen Fokussierung, der Gelfiltration, Gelchromatographie oder Ultrafiltration oder auch die Eigenschaft des $PP_{20}$, an schwach basische Ionenaustauscher gebunden und hiervon wieder eluiert werden zu können, verwendet werden.

Durch eine zweckmäßige Kombination der genannten Maßnahmen, die eine Anreicherung des $PP_{20}$ oder eine Abtrennung dieses Proteins von anderen Proteinen bewirken, kann das $PP_{20}$ isoliert werden.

Demzufolge ist ein Gegenstand der vorliegenden Erfindung in den einzelnen Schritten zur Anreicherung des $PP_{20}$ und in dem durch Kombination der Maßnahmen zur Anreicherung sich ergebenden Verfahren zur Reinigung des $PP_{20}$ zu sehen.

Die im Beispiel angegebenen Schritte zur Anreicherung und Isolierung von $PP_{20}$ sind keinesfalls alle

zwingend und müssen auch nicht in der dort beschriebenen Reihenfolge durchgeführt werden.

Zur Immunadsorption könnte man direkt den Extrakt aus menschlichen Plazenten einsetzen. Da die Konzentration von $PP_{20}$ im Plazentenextrakt aber relativ gering ist, ist es zweckmäßig, durch eine Vorfraktionierung des Extraktes das Protein $PP_{20}$ erst spezifisch anzureichern mit Hilfe von Methoden, die sich zur Fraktionierung von Proteinen in größerem Maßstab eignen; zum Beispiel durch fraktionierte Fällung mit Neutralsalzen oder organischen Kationen, durch Gelfiltration oder durch Ionenaustauschchromatographie. Auch der Schritt der Immunadsorption könnte durch Anwendung anderer Trennmethoden, zum Beispiel durch präparative Elektrophorese oder isoelektrische Fokussierung ersetzt werden.

Zur Hochreinigung von $PP_{20}$ im Endstadium der Isolierung haben sich Gelfiltration an Acrylamidagarose AcA 34 und inverse Immunadsorption als brauchbar erwiesen.

Aus einer ausgewachsenen menschlichen Plazenta (600 g) lassen sich mit physiologischer Salzlösung im Durchschnitt 0,5 mg dieses Proteins extrahieren. Außer in der Plazenta konnte $PP_{20}$ beim Menschen nur noch in Extrakten aus der Milz nachgewiesen werden.

Andere Organe des Menschen (zum Beispiel Herz, Lunge, Haut, Magen, Niere, Uterus, Leber, Nebenniere, Colon und Blase) enthalten dieses Protein nicht oder in wesentlich geringerer Konzentration. Auch im Serum oder in anderen Körperflüssigkeiten des Menschen kommt $PP_{20}$ normalerweise nicht oder nur in Spuren (unterhalb 1 mg/l) vor.

Zum Nachweis und zur Bestimmung des $PP_{20}$ etwa in einer Fraktion aus einer Trennoperation können neben den angegebenen Parametern auch immunchemische Methoden dienen, da $PP_{20}$ antigene Eigenschaften hat. Bei der Immunisierung von Tieren mit diesem Protein werden spezifische Antikörper gebildet.

Ein für diesen Zweck brauchbares Antiserum kann folgendermaßen gewonnen werden: Bei der Fraktionierung des Plazentaextraktes mit einer Acridinbase und Ammoniumsulfat nach Bohn (Arch. Gynäk., 210 (1971), 440-457) wird. $PP_{20}$ hauptsächlich in der Plazentafraktion II gefunden. Trennt man diese Fraktion durch Gelfiltration von Sephadex® G-150 weiter auf, so erscheint $PP_{20}$ im Bereich der niedermolekularen Proteine (Bereich 30 000-70 000). Versetzt man diese Fraktion nach Dialyse gegen einen 0,01 molaren Tris-HCL Puffer (pH 7,0) mit einer genügenden Menge Bentonit A, so werden die meisten Proteine an Bentonit adsorbiert, während $PP_{20}$ im Überstand gelöst bleibt. Durch Immunisieren von Kaninchen mit diesem Überstand erhält man ein polyvalentes Antiserum, das zur Hauptsache Antikörper gegen $PP_{20}$ enthält. Dieses Antiserum kann durch Absorption mit normalem menschlichem Serum und mit bestimmten Plazentaproteinen, wie alpha 1-Fetoprotein (AFP) und Ferritin ($PP_{20}$) gegen das Antigen $PP_{20}$ weitgehend spezifisch gemacht werden.

Ein solches Antiserum kann einerseits zum immunologischen Nachweis des $PP_{20}$, andererseits zur Herstellung eines Immunadsorbens dienen, das zur Anreicherung und Isolierung von $PP_{20}$ eingesetzt werden kann.

Mit Hilfe des nach der vorliegenden Anmeldung gewonnenen hochgereinigten $PP_{20}$ lassen sich durch Immunisieren von Tieren nach bekannten Methoden monospezifische Antiseren herstellen.

Abbildung 1a zeigt die immunologische Reaktion von $PP_{20}$ mit einem spezifischen Antiserum vom Kaninchen nach Auftrennung im elektrischen Feld in Agar-haltigem Gel.

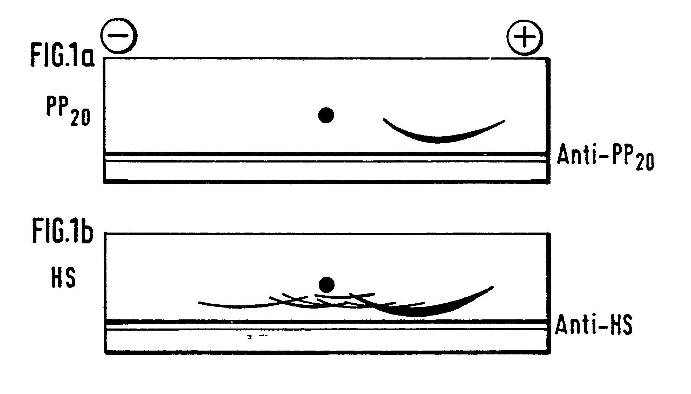

Abbildung 1b bringt zum Vergleich dazu die Auftrennung der Proteine des Serums, sichtbar gemacht durch deren Immunreaktion mit einem Antiserum vom Kaninchen gegen Humanserum (HS).

Zum immunologischen Nachweis von $PP_{20}$ können auch die Geldiffusionstechnik nach Ouchterlony (vgl. Schultze and Heremans, Molecular Biology of Human Proteins, Vol. 1, p. 134) oder wenn notwendig auch empfindlichere Methoden wie Radioimmunoassays oder Enzymimmunoassays herangezogen werden.

Der Nachweis und die Bestimmung von $PP_{20}$ haben diagnostische Bedeutung: $PP_{20}$ ist ein Protein, das außer in der Plazenta nur noch in der Milz in größerer Konzentration vorzukommen scheint. Bei Schwangerschaft, aber auch bei Erkrankungen von Organen, beispielsweise bei Tumoren, kann die Konzentration dieses Gewebsproteins im Serum oder in anderen Körperflüssigkeiten der Patienten über die Norm ansteigen. $PP_{20}$ kann daher als Marker zur Überwachung einer Schwangerschaft oder zum Nachweis und zur Verlaufskontrolle von Erkrankungen eingesetzt werden.

$PP_{20}$ kann also zur Herstellung spezifischer Antikörper gegen dieses Protein sowie zum Aufbau von immunchemischen Methoden (zum Beispiel Radioimmunoassay, Enzymimmunoassay) zum Nachweis und zur Bestimmung dieses Proteins verwendet werden.

Die Erfindung wird am nachstehenden Beispiel erläutert.

**Beispiel**

A) Extraktion der Plazenten und Fraktionierung des Extraktes mit einer Acridinbase und Ammoniumsulfat

1 000 kg tiefgefrorene menschliche Plazenten wurden im Schneidmischer zerkleinert und mit 1 000 Liter einer 0,4 %-igen (g/100 ml) Kochsalzlösung extrahiert. Der Extrakt wurde nach Abtrennung des Geweberückstandes durch Zentrifugation mit 20 %-iger (g/100 ml) Essigsäure auf pH 6,0 eingestellt und unter Rühren mit 200 Liter einer 3 %-igen (g/100 ml) Lösung von 2-Äthoxy-6,9-Diaminoacridin-Lactat (Rivanol®, Hoechst AG) versetzt. Der Niederschlag wurde durch Zentrifugation abgetrennt, mit 500 Liter einer 2,5 %-igen (g/100 ml) NaCl-Lösung versetzt und 4 Stunden gerührt. Das abgeschiedene Chlorid des 2-Äthoxy-6,9-Diaminoacridins wurde abzentrifugiert. Aus dem Überstand wurde ein Teil der Begleitproteine durch Zusatz von 25 % (g/100 ml) Ammoniumsulfat abgeschieden. Das Protein PP$_{20}$ blieb zur Hauptsache in Lösung. Es wurde daraus aurch weitere Zugabe von Ammoniumsulfat (20 g/100 ml) präzipitiert und dann abzentrifugiert. Es wurden dabei etwa 3 kg einer feuchten Paste, die im Nachfolgenden als Fraktion A bezeichnet wird, erhalten.

B) Gelfiltration an Sephadex® G-150

500 g der Fraktion A wurden in Wasser gelöst, zur Entfernung des noch vorhandenen Rivanols ® mit etwa 2,5 g Bentonit A (Fa. Erbslöh & Co., Geisenheim/Rh.) versetzt und nach Zentrifugation gegen einen 0,01 mol/l Tris-HCl-Puffer (pH 8,0), der 0,05 % (g/100 ml) NaN$_3$ enthielt (Pufferlösung I), dialysiert. Die zurückbleibende Lösung wurde auf eine mit Sephadex® G-150 gefüllte Säule (20 x 100 cm) aufgetragen und mit Pufferlösung I eluiert. Die Eluate mit den niedermolekularen Proteinen (MG 30 000 bis 70 000) wurden vereinigt und zur Fällung der Proteine mit 45 g/100 ml Ammoniumsulfat versetzt. Der Niederschlag wurde abzentrifugiert (Fraktion B).

C) Anreicherung von PP$_{20}$ durch Immunadsorption

1) Herstellung eines Antiserums gegen PP$_{20}$

100 ml einer etwa 1 g/100 ml Proteine enthaltenden Lösung der Fraktion B in Pufferlösung I wurden mit 15 g Bentonit A (Erbslöh & Co., Geisenheim/Rh.) versetzt, 30 Minuten gerührt und dann zentrifugiert. Die Hauptmenge der in der Lösung enthaltenen Proteine wurde dabei durch Adsorption an Bentonit entfernt, während PP$_{20}$ im Überstand blieb. Der Überstand wurde dann mittels eines Ultrafilters auf 6,5 ml eingeengt, gegen physiologische NaCl-Lösung dialysiert und zur Immunisierung von 5 Kaninchen eingesetzt. Die dabei gewonnenen Antiseren enthielten Antikörper sowohl gegen PP$_{20}$ als auch gegen einige andere im Überstand verbliebenen Proteine wie Albumin, Praealbumin, alpha 1-Fetoprotein (AFP) und Ferritin. Durch Absorption mit Normalserum, AFP und einem aus Plazenten isolierten Ferritin wurde das Antiserum gegen PP$_{20}$ weitgehend spezifisch gemacht.

2) Herstellung des Immunadsorbens

300 ml des Anti-PP$_{20}$-Serums vom Kaninchen, dessen Gewinnung unter C 1) beschrieben ist, wurden gegen 0,02 molaren Phosphatpuffer (pH 7,0) dialysiert und zur Abtrennung der Immunglobuline an DEAE-Zellulose chromatographiert. Die Immunglobulinfraktion (1,77 g Protein) wurde dann mit 178 g besonders gereinigter Agarose in Kugelform (Sepharose® der Pharmacia, Uppsala, Schweden), die mit 22,2 g Bromcyan aktiviert worden war, umgesetzt und so kovalent an einen Träger gebunden. Das Verfahren wurde beschrieben von Axen R., Porath J., Ernbach S., Nature 214, 1302 (1967). Mit Hilfe eines auf diese Weise hergestellten Immunoadsorbens konnte PP$_{20}$ aus den mit PP$_{20}$ angereicherten Plazentaextraktfraktionen isoliert werden.

3) Durchführung der Immunadsorption

Das Immunadsorbens wurde in einen 0,1 molaren Tris-HCl-Puffer von pH 8, der 1 mol/l NaCl und 0,1 g/100 ml Natriumacid enthielt (Pufferlösung II), suspendiert, in eine Chromatographiesäule (4,5 x 14 cm) gefüllt und mit Pufferlösung II nachgespült.

Dann wurde die in Wasser gelöste und gegen Pufferlösung II dialysierte Fraktion B auf die Säule aufgetragen, wobei PP$_{20}$ immunadsorptiv gebunden wurde. Die Säule wurde gründlich mit Puffer II gespült. Anschließend ist das adsorbierte Protein mit etwa 600 ml 6-molarer Harnstofflösung von der Säule eluiert worden. Die PP$_{20}$-haltigen Eluate wurden gegen Pufferlösung II dialysiert und mit einem Ultrafilter auf etwa 10 ml eingeengt. Die Ausbeute betrug pro Adsorption etwa 6 mg PP$_{20}$. Das Adsorbens in der Säule wurde unmittelbar nach der Elution von PP$_{20}$ mit Pufferlösung gründlich gewaschen. Es konnte dann erneut zur immunadsorptiven Bindung von PP$_{20}$ eingesetzt werden.

D) Hochreinigung von PP$_{20}$

Das durch Immunadsorption gewonnene Protein war häufig durch geringe Mengen unspezifisch gebundener Serumproteine und anderer Plazenta-Gewebsproteine verunreinigt. Die Abtrennung dieser Begleitproteine gelang zum Teil durch Gelfiltration an Acrylamidagarose AcA 34. Die restlichen Verunreinigungen wurden dann durch inverse oder negative Immunadsorption, das heißt mit Hilfe von trägergebundenen Antikörpern gegen die noch als Verunreinigung vorliegenden Proteine (PP$_9$, PP$_{11}$, PP$_{16}$, AFP und Serumproteine) entfernt.

**Patentansprüche**

1. Protein PP$_{20}$, gekennzeichnet durch
a) eine elektrophoretische Beweglichkeit bei pH 8,6, die etwas größer ist als die von Albumin,
b) einen isoektrischen Punkt von 4,65 ± 0,1,
c) einen Sedimentationskoeffizienten $s_{20,w}$ von 4,1 ± 0,1 S,
d) ein im Natriumdodecylsulfat (SDS)-haltigen Polyacrylamidgel electrophoretisch bestimmtes Molekulargewicht von 50 000 ± 10 000, wobei die Moleküle von PP$_{20}$ aus anscheinend identischen Untereinheiten, die ein Molekulargewicht von 27 000 ± 3 000 haben und nicht-kovalent zusammengehalten werden, aufgebaut sind,
e) einen Extinktionskoeffizienten $E^{1\%}_{1cm}$ (280 nm) von 9,5 ± 0,6,
f) einen Kohlenhydratanteil von 3,0 ± 1,3 %, darunter Mannose, 0,14 ± 0,05 %, Fucose 0,13 ± 0,05 %, Galaktose 0,61 ± 0,2 %, Glukose 0,39 ± 0,2 %, N-Acetyl-Glukosamin 0,95 ± 0,3 %, N-Acetyl-Galaktosamin 0,18 ± 0,1 %, N-Acetyl-Neuraminsäure 0,6 ± 0,4 % und
g) die folgende Aminosäurenzusammensetzung:

| Aminosäure | (Reste pro 1,00 Reste) Mol-% | Variations- koeffizient |
|---|---|---|
| Lysin | | |
| Histidin | | |
| Arginin | | |
| Asparaginsäure | | |
| Threonin | | |
| Serin | | |
| Glutaminsäure | | |
| Prolin | | |
| Glycin | | |
| Alanin | | |
| Cystin 1/2 | | |
| Valin | | |
| Methionin | | |
| Isoleucin | | |
| Leucin | | |
| Tyrosin | | |
| Phenylalanin | | |
| Tryptophan | | |

2. Verfahren zur Gewinnung des Proteins $PP_{20}$ nach Anspruch 1, dadurch gekennzeichnet, daß ein aus Plazenten mit einer Verdünnten Salz- oder Pufferlösung gewonnen Extrakt zwei oder mehr der folgenden Maßnahmen unterworfen wird:

a) Fällung des Proteins $PP_{20}$ mit Ammoniumsulfat im pH-Bereich Von 5 bis 8 und bei 30 - 70 % Sättigung;

b) Fällung des Proteins $PP_{20}$ mit einer wasserlöslichen Acridinbase bei einem pH-Wert zwischen 4 und 7 und einer Konzentration der Base von 0,2 - 0,4 g/100 ml;

c) präparativ Zonenelektrophorese, wobei die Proteinfraktion, die bei pH 8,6 etwas schneller als Albumin wandert, gewonnen wird

d) Gelfiltration oder Ultrafiltration, wobei Proteine im Molekulargewichtsbereich von 30 000 bis 70 000 gewonnen werden;

e) isoelektrische Fokussierung, wobei die Proteine im pH-Bereich von 4,55 bis 4,75 gewonnen werden;

f) Adsorption von Begleitproteine an Bentonit, wobei $PP_{20}$ in Lösung bleibt;

g) Adsorption des Proteins $PP_{20}$ an einem schwach basischen Ionenaustauscher und Elution dieses Proteins;

h) immunadsorptive Anreicherung des Proteins $PP_{20}$.

3. Verwendung des Proteins $PP_{20}$ nach Anspruch 1 zur Gewinnung eines Antiserums und zum Aufbau von immunchemischen Methoden zum Nachweis und zur Bestimmung dieses Proteins, um eine Schwangerschaft zu überwachen oder zum Nachweis oder zur Verlaufskontrolle von Erkrankungen.

**Claims**

1. The protein $PP_{20}$ which has the following characteristics:

a) an electrophoretic mobility at pH 8.6 which is somewhat larger than that of albumin,

b) an isoelectric point of $4.65 \pm 0.1$

c) a sedimentation coefficient $s_{20,w}$ of $4.1 \pm 0.1$ S,

d) a molecular weight determined by electrophoresis in sodium dodecyl sulfate (SDS)-containing polyacrylamide gel of $50,000 \pm 10,000$, the molecules of $PP_{20}$ being composed of apparently identical subunits which have a molecular weight of $27,000 \pm 3,000$ and are held together non-covalently,

e) an extinction coefficient $E^{1\%}_{1cm}$ (280 nm) of $9.5 \pm 0.6$

f) a carbohydrate content of $3.0 \pm 1.3$ %, including mannose $0.14 \pm 0.05$ %, fucose $0.13 \pm 0.05$ %, galactose $0.61 \pm 0.2$ %, glucose $0.39 \pm 0.2$ %, N-acetylglucosamine $0.95 \pm 0.3$ %, N-acethylgalactosamine $0.18 \pm 1$ %, N-acetylneuraminic acid $0.6 \pm 0.4$ %, and

9) the following aminoacid composition:

| Aminoacid | (Residues per 100 residues) mole-% | coefficient of variation |
|-----------|-----------|-----------|
| Lysine | 5.36 | 0.26 |
| Histidine | 3.10 | 7.30 |
| Arginine | 3.39 | 0.63 |
| Aspartic acid | 11.45 | 1.05 |
| Threonine | 8.63 | 1.31 |
| Serine | 3.70 | 1.34 |
| Glutamic acid | 9.65 | 1.61 |
| Proline | 5.64 | 6.40 |
| Glycine | 6.96 | 1.12 |
| Alanine | 4.76 | 1.49 |
| Cystine 1/2 | 2.86 | 2.47 |
| Valine | 6.22 | 0.23 |
| Methionine | 1.87 | 1.51 |
| Isoleucine | 5.76 | 3.44 |
| Leucine | 11.76 | 2.65 |
| Tyrosine | 2.55 | 3.33 |
| Phenylalanine | 4.12 | 1.55 |
| Tryptophan | 2.17 | 1.30 |

2. A process for obtaining the protein $PP_{20}$ as claimed in claim 1, which comprises subjecting an extract obtained from placentae using a dilute salt or buffer solution to two or more of the following measures:

a) precipitation of the protein $PP_{20}$ with ammonium sulfate in the pH range from 5 to 8 and at 30 - 70 % saturation;

b) precipitation of the protein $PP_{20}$ with a water-soluble acridine base at a pH between 4 and 7 and a concentration of the base of 0.2 - 0.4 g/100 ml;

FIG.1a

PP$_{20}$

Anti-PP$_{20}$

FIG.1b

HS

Anti-HS